(19) Europäisches Patentamt
European Patent Office
Office européen des brevets

(11) Veröffentlichungsnummer: **0 244 613 B1**

(12) # EUROPÄISCHE PATENTSCHRIFT

(45) Veröffentlichungstag der Patentschrift: **21.06.89**

(51) Int. Cl.⁴: **C07C 87/34,** C07C 85/24

(21) Anmeldenummer: **87104215.6**

(22) Anmeldetag: **21.03.87**

(54) **Trifluormethylgruppen enthaltende Cyclopropylamine.**

(30) Priorität: **04.04.86 DE 3611196**

(43) Veröffentlichungstag der Anmeldung: **11.11.87 Patentblatt 87/46**

(45) Bekanntmachung des Hinweises auf die Patenterteilung: **21.06.89 Patentblatt 89/25**

(84) Benannte Vertragsstaaten: **BE CH DE FR GB IT LI NL**

(56) Entgegenhaltungen:
**THE JOURNAL OF ORGANIC CHEMISTRY, Band 27, 1962, Easton. M.S. RAASCH: "The chemistry of sulfur tetrafluoride. IX. Reaction with amino acids in hydrogen fluoride", Seiten 1406-1409**
**CHEMICAL ABSTRACTS, Band 88, Nr. 25, 19. Juni 1978, Columbus, Ohio, USA. D.H. RICH et al.: "A convenient synthesis of the amino acid, 1-aminocyclopropane-1-carboxylic acid", Seite 702, Zusammenfassung Nr 190 132g**
**Synthesis 1978, (1), 46**

(73) Patentinhaber: **BAYER AG, D-5090 Leverkusen 1 Bayerwerk(DE)**

(72) Erfinder: **Baasner, Bernd, Dr., Hambergerstrasse 27 d, D-5090 Leverkusen 3(DE)**

EP 0 244 613 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Anmeldung betrifft neue Trifluormethylgruppen enthaltende Cyclopropylamine und Verfahren zu ihrer Herstellung.

Es wurden neue Trifluormethylgruppen enthaltende Cyclopropylamine der Formel (I)

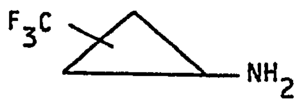

(I)

gefunden.

Es wurde weiterhin gefunden, daß man die Cyclopropylamine der Formel (I)

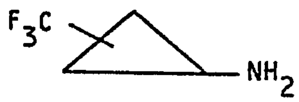

(I)

herstellen kann, indem man Cyclopropylcarbonsäureamine der allgemeinen Formel (II)

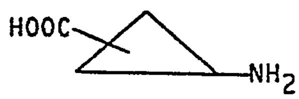

(II)

mit Schwefeltetrafluorid in wasserfreiem Fluorwasserstoff umsetzt.

Die neuen Verbindungen der allgemeinen Formel (I) eignen sich als wertvolle Zwischenprodukte für Synthesen in der organischen Chemie. Insbesondere geeignet sind die neuen Verbindungen der Formel (I) für die Herstellung von neuen Benzoesäureamiden, die insektizide Wirkung aufweisen. Folgende Synthese mag hier beispielhaft aufgeführt werden:

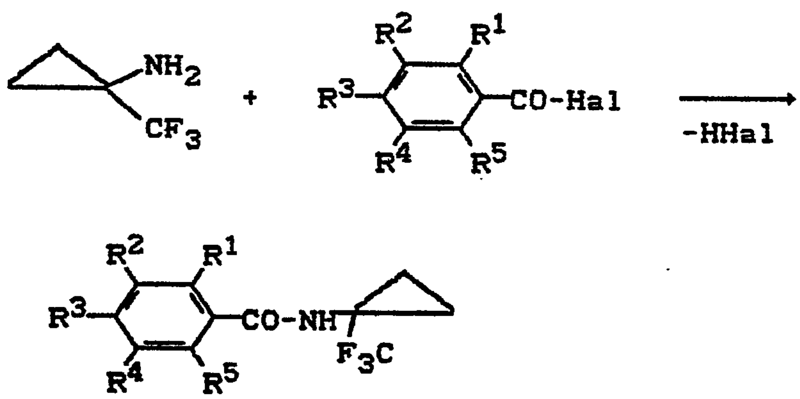


wobei die Substituenten $R^1$, $R^2$, $R^3$, $R^4$ und $R^5$ gleich oder verschieden sein können und für Wasserstoff, Halogen, Nitro, Alkyl, Halogenalkyl, Halogenalkoxy oder Halogenalkylthio stehen.

Folgende Verbindungen der allgemeinen Formel (I) kommen in Frage:

1-Trifluormethyl-cyclopropylamin

2-Trifluormethyl-cyclopropylamin.

Verwendet man 1-Amino-cyclopropancarbonsäure als Ausgangsprodukt, so kann die Umsetzung wie folgt dargestellt werden:

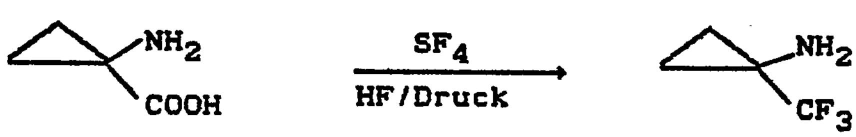


Die Umsetzung der Cyclopropancarbonsäuren der Formel (II) mit Schwefeltetrafluorid zur Einführung der Trifluormethylgruppe erfolgt nach einem an sich bekannten Verfahren in wasserfreiem Fluorwasserstoff (siehe dazu Journal of Organic Chemistry 27, 1406 (1962)).

Dazu werden die Aminocarbonsäuren der allgemeinen Formel (II) in einem Stahlrührautoklaven in Fluorwasserstoff mit Schwefeltetrafluorid bei 100° bis 180°, bevorzugt bei 120° bis 160° unter Eigendruck etwa 4 bis 12 Stunden, bevorzugt 6 bis 10 Stunden lang umgesetzt. Pro Mol Aminocarbonsäure werden 2 bis 10 Mol, bevorzugt 2 bis 5, besonders bevorzugt 2 bis 3 Mol Schwefeltetrafluorid sowie 3 bis 200, bevorzugt 3 bis 100, ganz besonders bevorzugt 3 bis 60 Mol Fluorwasserstoff eingesetzt. Zur Aufarbeitung des Umsetzungsgemisches werden die üblicherweise angewandten Verfahren benutzt. So kann man die flüchtigen Bestandteile abdestillieren, das Rohprodukt mit wäßriger Alkalilauge alkalisch stellen und das Produkt daraus durch Destillation, Extraktion oder Wasserdampfdestillation abtrennen.

Wird beispielsweise das Amin durch Wasserdampfdestillation abgetrennt, kann das Produkt aus dem Destillat, sofern es sich als zweite Phase abscheidet, durch Phasentrennung isoliert werden. Das Destillat kann aber auch mit Salzsäure angesäuert werden. Durch Einengen bis zur Trockne wird dann das Hydrochlorid des Amins der allgemeinen Formel (I) in reiner Form erhalten. Daraus kann das freie Amin der allgemeinen Formel (I) durch Behandlung mit einer Lauge zur Neutralisation der Salzsäure aus dem Hydrochlorid gewonnen werden. Das Amin wird durch Abdestillieren aus diesem Rohgemisch in reiner Form gewonnen. Es ist aber auch möglich, das Amin der allgemeinen Formel (I) aus diesem Rohgemisch mit einem inerten organischen Lösungsmittel zu extraktieren und das Amin aus diesem Extrakt durch Destillation zu isolieren.

Die Cyclopropancarbonsäureamine der allgemeinen Formel (II) sind bekannt (z.B. J. Chem. Soc. 1960, 2119-2132; ibid. 1962, 3977-3980; Synthesis 1978, 46; Coll. Czech. Chem. Comm. 47, 2291-2305/(1982)) oder können nach bekanntem Verfahren hergestellt werden.

Herstellungsbeispiele

Beispiel 1

1-Trifluormethyl-cyclopropylamin-hydrochlorid

40 g (0,4 Mol) Cyclopropyl-1-aminocarbonsäure werden mit 100 g Schwefeltetrafluorid und 50 ml HF 8 Stunden lang in einem V4A-Rührautoklaven bei 120° C unter Eigendruck (30 → 25 bar) umgesetzt. Nach Abdestillieren der flüchtigen Bestandteile wird mit 45 %iger Natronlauge alkalisch gestellt und das Produkt anschließend durch Wasserdampfdestillation abgetrennt. Das Wasserdampfdestillat wird mit konzentrierter Salzsäure sauer gestellt. Nach Einengen und Trocknung werden 40 g (62 %) 1-Trifluormethylcyclopropylamin als Hydrochlorid (Fp. : >260°C) isoliert.

Beispiel 2

1-Trifluormethyl-cyclopropylamin

Zu 32,4 g (0,2 Mol) 1-Trifluormethylcyclopropylamin-hydrochlorid aus Beispiel 1 werden 44 g (0,22 Mol) 20 %ige wäßrige Natronlauge bei 80° -90° C Ölbadtemperatur in ca. 15 Minuten getropft. Gleichzeitig wird das freigesetzte Amin abdestilliert. Nach Trocknen über Magnesiumsulfat wird redestilliert. Man erhält an Produkt

22,2 g (89 %) 1-Trifluormethyl-cyclopropylamin, Kp.: 50 bis 52° C, $n_D^{20}$: 1.3483

## Patentansprüche

1. Cyclopropylamine der Formal (I)

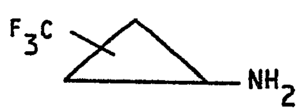

(I)

2. 1-Trifluormethyl-cyclopropylamin.
3. 2-Trifluormethyl-cyclopropylamin.
4. Verfahren zur Herstellung von Cyclopropylaminen der Formel (I)

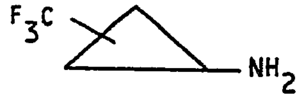

(I)

dadurch gekennzeichnet, daß man Cyclopropylcarbonsäureamine der Formel (II)

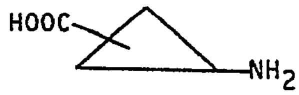

(II)

mit Schwefeltetrafluorid in wasserfreiem Fluorwasserstoff umsetzt und die Reaktionsprodukte nach üblichen Methoden aufarbeitet.

**Claims**

1. Cyclopropylamines of the formule (1)

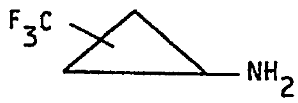

(I)

2. 1.Trifluoromethyl-cyclopropylamine.
3. 2-Trifluoromethyl-cyclopropylamine.
4. Process for the preparation of cyclopropylamines of the formula (1)

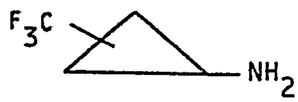

(I)

characterized in that cyclopropylcarboxylic acid amines of the formula (II)

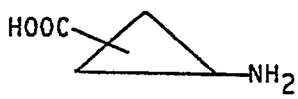

(II)

are reacted with sulphur tetrafluoride in anhydrous hydrogen fluoride and the reaction products are worked up by customary methods.

**Revendications**

1. Les cyclopropylamines de formule (1)

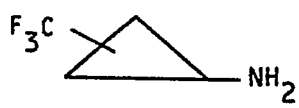

(I)

2. La 1-trifluorométhylcyclopropylamine.
3. La 2-trifluorométhylcyclopropylamine.
4. Procédé pour la fabrication de cyclopropylamines de formule (1)

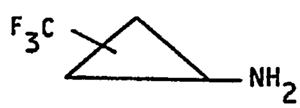

(I)

caractérisé en ce que l'on fait réagir des acides aminocyclopropyl-carboxyliques de formule (II)

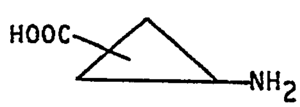

(II)

avec le tétrafluorure de soufre dans le fluorure d'hydrogène anhydre et on traite les produits de réaction par des techniques habituelles.